# EUROPEAN PATENT SPECIFICATION

(11) **EP 1 245 275 B1**
(45) Date of publication and mention of the grant of the patent: **26.07.2006**
(21) Application number: 01127358.8
(22) Date of filing: 21.11.2001
(51) Int. Cl.: B01J 20/22, A61L 9/01, A61L 9/012

(54) **Odour-absorbing compound in particular for refrigerators**
Geruchsabsorbierendes Mittel, insbesondere für Kühlschränke
Composé absorbant les odeurs en particulier pour refrigerateur

(30) Priority: 29.03.2001 IT MI010667
(43) Date of publication of application: 02.10.2002
(73) Proprietor: TAVOLA S.p.A., 20141 Milano (IT)
(72) Inventor: Ferruccio, Varana, 20100 Milano (IT)
(74) Representative: La Ciura, Salvatore

(56) References cited:
- DE-A- 19 937 871
- GB-A- 1 241 914
- GB-A- 1 282 889
- GB-A- 2 096 891
- US-A- 6 139 880
- DATABASE WPI Section Ch, Week 198946 Derwent Publications Ltd., London, GB; Class D22, AN 1989-336054 XP002224740 & JP 01 250262 A (NIPPON ZEON KK), 5 October 1989 (1989-10-05)
- DATABASE WPI Section Ch, Week 199003 Derwent Publications Ltd., London, GB; Class D22, AN 1990-020007 XP002224741 & JP 01 300954 A (LION CORP), 5 December 1989 (1989-12-05)
- DATABASE WPI Section Ch, Week 198644 Derwent Publications Ltd., London, GB; Class A84, AN 1986-289224 XP002224742 & JP 61 213053 A (SEKISUI CHEM IND CO LTD) , 22 September 1986 (1986-09-22)
- DATABASE WPI Section Ch, Week 198830 Derwent Publications Ltd., London, GB; Class D22, AN 1988-210073 XP002224743 & JP 63 147538 A (NITTO ELECTRIC IND CO), 20 June 1988 (1988-06-20)
- DATABASE WPI Section Ch, Week 197717 Derwent Publications Ltd., London, GB; Class A97, AN 1977-29980Y XP002224744 & JP 52 034934 A (JAPAN SYNTHETIC RUBBER CO LTD), 17 March 1977 (1977-03-17)

## Description

This invention relates to an odour-absorbing compound fit in particular to deodorize small closed rooms such as refrigerators.

The compound according to the invention looks like a gel and therefore it can be put in containers of the most different shapes, being effective in absorbing even strong smells with a long-lasting action.

Furthermore, putting the gel into a container, being at least partially transparent, it is possible to check the remaining quantity of the product and then to learn how long it is going to be effective yet.

The compound according to the invention is ricinoleate-zinc-based, being this product already used as a deodorant, but in other fields and in different compounds.

The components being used in this invention are all natural components so that they can be left in contact with food without any problem.

The compound comprises the following components:
- natural gel extracted from red algae;
- ricinoleate-zinc functioning as an odour-neutralizing agent;
- polyethoxylated hydrogenated castor oil as a solubilizing agent, in addition to
- colourings, preservatives, demineralized water and any products fit to give the mixture an unpleasant taste in order to avoid ingestion, even unintentional, by children.

A gel-shaped compound is obtained which can be easily packed or put inside different containers to exert its odour-absorbing action in time.

Different compounds exist fitted to eliminate food smell from refrigerators, but they usually are detergents or similar products aimed at washing the inside of the refrigerator after the content being removed.

Besides that, specific products are known consisting of deodorants or the like releasing a fragrance which is added to the odours being in the fridge in order to cover them.

Products have been also proposed aimed at absorbing food odours, but these systems are based on the use of activated carbons functioning as a filtering unit, having a limited effectiveness and not letting the user have information about the remaining duration of the product.

US 6.139.880 describes gel cosmetic odour-absorbing or deodorant compositions, which comprises an amount of zinc ricinoleate in a gel base.

GB 1.282.889 too, discloses a deodorant composition which comprises, as active ingredient, a zinc salt of an unsaturated aliphatic hydroxycarboxylic acid.

In this field this invention is hereby presented, which offers a deodorizing compound for refrigerators able to absorb odours coming from food, being based on the use of ricinoleate-zinc blended in a gel-shaped base mixture.

The compound thus obtained can be easily put inside containers of the most proper shape and size without any difficulty, exerting afterwards an effective odour-absorbing action lasting some weeks.

This invention will be hereby described in detail by way of a non limitative example with reference to the following:

### EXAMPLE

The odour-absorbing compound according to the invention has the following content, in which the quantities of the different components are shown according to the weight:
- carrageenan gel (gel extracted from red algae), from 1 to 5 per cent;
- isothiozaolinone mixture, functioning as a preservative, in a quantity from 0,02 up to 0,5 per cent;
- ricinoleate-zinc (active ingredient) in a quantity from 0,05 up to 1 per cent;
- hydrogenated castor oil 40 EO, functioning as a solubilizing agent in a quantity comprised between 0,5 and 5 per cent;
- DTMP sodium salt 32-per-cent solution functioning as a sequestrant preventing zinc oxidation, in a quantity from 0,03 up to 0,1 per cent ;
- benzoate denatonium (anti-ingestion) in a quantity from 0,001 up to 0,01 per cent;
- blue E131 (food colouring) in a quantity from 0,0001 up to 0,001 per cent;
- yellow tartrazine E102 (food colouring) in a quantity from 0,0001 up to 0,001 per cent;
- as much demineralized water as needed up to 100.

The alga gel is available on sale, being made for example by the firm ROHM AND HAAS under the brand GUMGEL M174; the isothiozaolinone mixture is available under the brand KATHON CG being made as well by the firm ROHM AND HAAS; the ricinoleate-zinc is sold for example by the firm GOLDSCHMIDT under the brand TEGO SORB; the hydrogenated castor oil is available under the brand CREMOPHOR RH40 of the company BASF; the sequestrant DTMP sodium salt is available under the brand SEQUION 40 Na 32; the benzoate denatonium is available on sale under the brand BITREX.

Different tests carried out by the applicant enabled to point out that optimal results can be reached with the following composition:
- carrageenan gel: 2,5 per cent;
- isothiozaolinone mixture: 0,05 per cent;
- ricinoleate-zinc: 0,2 per cent;
- polyethoxylated hydrogenated castor oil 40 EO: 2 per cent;
- SEQUION 40 na 32: 0,06 per cent;
- BITREX: 0,002 per cent;
- blue E131 colouring: 0,000375 per cent;
- yellow tartrazine E102 colouring: 0,000375 per cent;
- demineralized water: 95,1872 per cent.

The preparation of the compound is carried out as follows.

A mixer is filled with the whole quantity of mineralized water at ambient temperature and the GUMGEL M174 is added slowly stirring forcefully.

The stirring is to be continued at ambient temperature until the jelling agent is completely dispersed, after that, after checking the absence of lumps, the heating is started.

The temperature is brought up to 60-65°C under stirring and it is kept for a time of 10-20 minutes.

Then the isothiozaolinone mixture is added and mixed carefully; the colouring is then added always mixing carefully until it is completely dispersed.

The ethoxylated hydrogenated castor oil 40E0 and the ricinoleate zinc are mixed together in a separated mixer.

After checking the right temperature has been reached, the mixture of ricinoleate zinc and solubilizing agent is poured into the main mixer under continuous stirring, being the mixing continued until a homogeneous distribution of the mass is obtained.

The sequestrant is to be added in the end mixing carefully until the sodium salt is completely dissolved in the solution.

Finally BITREX is added always mixing till total dispersion, after that it is possible to have the product filtered into the final containers.

A gel-shaped compound is thus obtained fit to absorb effectively even strong smells such as fish smell or the like inside refrigerators, perfectly functioning even at low temperatures.

The active ingredient remains in suspension inside the gel and coming into contact with the air absorbs the odours.

As time goes by, the water being in the gel composition evaporates and the gel volume diminishes.

Therefore, using a container being at least partially transparent, it is possible to check the remaining quantity of the product and then its residual duration and effectiveness.

A skilled in the art may make changes and modifications as long as these are within the scope of the claimed invention.

## Claims

1. An odour-absorbing compound for refrigerators, **characterized in that** it comprises for the following components:
- carrageenan gel;
- isothiozaolinone mixture;
- ricinoleate zinc;
- polyethoxylated hydrogenated castor oil 40 EO;
- DTMP sodium salt;
- benzoate denatonium;
- any colourings;
- water.

2. Odour-absorbing compound for refrigerators according to claim 1, **characterized in that** it has the following composition, in which all the component percentages are in weight:
- carrageenan gel from 1 to 5 per cent;
- isothiozaolinone mixture from 0,02 up to 0,5 per cent;
- ricinoleate zinc (active ingredient) from 0,05 up to 1 per cent;
- hydrogenated castor oil 40 EO from o,5 up to 5 per cent;
- DTMP sodium salt 32-per-cent solution from 0,03 up to 0,1 per cent ;
- benzoate denatonium (anti-ingestion) from 0,001 up to 0,01 per cent;
- blue E131 (food colouring) from 0,0001 up to 0,001 per cent;
- yellow tartrazine E102 (food colouring) from 0,0001 up to 0,001 per cent;
- as much demineralized water as needed up to 100 per cent.

3. Odour-absorbing compound for refrigerators according to claim 1, charaterized by the following composition, in which the component percentages in weight:
- carrageenan gel 2,5 per cent;
- isothiozaolinone mixture 0,05 per cent;
- ricinoleate-zinc 0,2 per cent;
- polyethoxylated hydrogenated castor oil 40 EO 2 per cent;
- DTMP sodium salt solution 0,06 per cent;
- Benzoate Denatonium 0,002 per cent;
- blue E131 colouring 0,000375 per cent;
- yellow tartrazine E102 colouring 0,000375 per cent;
- demineralized water 95,1872 per cent.

4. Process for preparing an odour-absorbing compound for refrigerators according to claims from 1 to 3, **characterized in that** it provides for the following phases:
- mixing of carrageenan gel in demineralized water at ambient temperature till total dispersion of the jelling agent;
- increasing of the mixture temperature up to 60-65°;
- adding of the isothiozaolinone mixture;
- possible adding of colourings;
- preparation of a mixture of ethoxylated hydrogenated castor oil 40 EO and ricinoleate-zinc in a separated mixer;
- adding of this mixture to the mixture prepared and heated before;
- adding of the sequestrant;
- possible adding of an anti-ingestion product.

5. Odour-absorbent for refrigerators, **characterized in that** it comprises a composition according to any of the preceding claims from 1 to 3, contained in a container being at least partially transparent so as to enable to check the remaining quantity of the product.

## Patentansprüche

1. Geruchsabsorbierende Verbindung für Kühlschränke, **dadurch gekennzeichnet; daß** sie die folgenden Komponenten umfaßt:
- Carrageenangel;
- Isothiozaolinongemisch;
- Ricinoleatzink;
- polyethoxyliertes hydriertes Rizinusöl 40 EO;
- DTMP-Natriumsalz;
- Denatoniumbenzoat;
- irgendwelche Färbemittel;
- Wasser.

2. Geruchsabsorbierende Verbindung für Kühlschränke nach Anspruch 1, **dadurch gekennzeichnet, daß** sie die folgende Zusammensetzung aufweist, wobei alle Prozentangaben der Komponenten bezogen auf das Gewicht angegeben werden:
- Carrageenangel von 1 bis 5 %:
- Isothiozaolinongemisch von 0,02 bis zu 0,5 %;
- Ricinoleatziak (Wirkstoff) von 0,05 bis zu 1 %;
- hydriertes Rizinusöl 40 EO von 0,5 bis zu 5 %;
- DTMP-Natriumsalz 32%ige Lösung von 0,03 bis zu 0,1 %;
- Denatoniumbenzoat (gegen die Nahrungsaufnahme) von 0,001 bis zu 0,01 %;
- Blau E131 (Lebensmittelfarbstoff) von 0,0001 bis zu 0,001 %;
- gelbes Tartrazin E102 (Lebensmittelfarbstoff) von 0,0001 bis zu 0,001 %;
- soviel demineralisiertes Wasser wie notwendig auf 100 %.

3. Geruchsabsorbierende Verbindung für Kühlschränke nach Anspruch 1, **gekennzeichnet durch** die folgende Zusammensetzung, wobei die Prozentangaben der Komponenten bezogen auf das Gewicht angegeben werden:
- Carrageenangel 2,5 %;
- Isothiozaolinongemisch 0,05 %;
- Ricinoleatzink 0,2 %;
- polyethoxyliertes hydriertes Rizinusöl 40 EO 2 %;
- DTMP-Natriumsalzlösung 0,06 %;
- Denatoniumbenzoat 0,002 %;
- blauer E131-Farbstoff 0,000375 %;
- gelber Tartrazin-E102-Farbstoff 0,000375 %;
- demineralisiertes Wasser 95,1872 %.

4. Verfahren zur Herstellung einer geruchsabsorbierenden Verbindung für Kühlschränke nach den Ansprüchen 1 bis 3, **dadurch gekennzeichnet, daß** es die folgenden Phasen umfaßt:
- Mischen von Carrageenangel in demineralisierten Wasser bei Umgebungstemperatur bis zur vollständigen Dispersion des Geliermittels;
- Erhöhen der Gemischtemperatur auf bis zu 60 - 65 °C;
- Zugeben des Isothiozaolinongemisches;
- mögliche Zugabe von Farbstoffen;
- Herstellung eines Gemisches aus ethoxyliertem hydriertem Rizinusöl 40 EO und Ricinoleatzink in einem separaten Mischer;
- Zugeben dieses Gemisches zu dem Gemisch, das zuvor hergestellt und erhitzt wurde;
- Zugeben des Maskierungsmittels;
- mögliches Zugeben eines Produktes gegen die Nahrungsaufnahme.

5. Geruchsabsorptionsrnittel für Kühlschränke, **dadurch gekennzeichnet, daß** es eine Zusammensetzung nach einem der vorhergehenden Ansprüche 1 bis 3 umfaßt, die in einem Behälter enthalten ist, der zumindest teilweise transparent ist, um so die Restmenge des Produktes prüfen zu können.

## Revendications

1. Composé absorbant les odeurs pour réfrigérateurs, **caractérisé en ce qu'**il comprend les constituants suivants :
1. du gel de carrageenan ;
2. un mélange d'isothiozaolinone ;
3. du ricinoléate de zinc ;
4. de l'huile de ricin hydrogénée polyéthoxylée à 40 oxyde d'éthylène ;
5. du sel de sodium de DTMP ;
6. du benzoate de dénatonium ;
7. un colorant quelconque ;
8. de l'eau.

2. Composé absorbant les odeurs pour réfrigérateurs suivant la revendication 1, **caractérisé en ce qu'**il a la composition suivante dans laquelle tous les pourcentages des constituants sont donnés en poids :
- gel de carrageenan de 1 à 5 % ;
- mélange d'isothiozaolinone de 0,02 à 0,5 % ;
- ricinoléate de zinc (ingrédient actif) de 0,05 à 1 % ;
- l'huile ricin hydrogénée à 40 oxyde d'éthylène de 0,5 à 5 % ;
- sel de sodium de DTMP en solution à 32 % de 0,03 à 0,1 % ;
- benzoate de dénatonium (anti-ingestion) de 0,001 à 0,01 % ;
- bleu E131 (colorant alimentaire) de 0,0001 à 0,001 % ;
- tartrazine jaune E102 (colorant alimentaire) de 0,0001 à 0,001 % ;
- autant d'eau déminéralisée qu'il en faut pour compléter à 100 %.

3. Composé absorbant les odeurs pour réfrigérateurs suivant la revendication 1, **caractérisé par** la composition suivante dans laquelle les pourcentages des constituants sont donnés en poids :
- gel de carrageenan 2,5 % ;
- mélange d'isothiozaolinone 0,05 % ;
- ricinoléate de zinc 0,2 % ;
- l'huile ricin hydrogénée polyéthoxylée à 40 oxyde d'éthylène 2 % ;
- sel de sodium de DTMP en solution 0,06 % ;
- benzoate de dénatonium 0,002 % ;
- bleu E131 colorant 0,000375 % ;
- tartrazine jaune E102 colorant de 0,000375 % ;
- eau déminéralisée 95,1872 %.

4. Procédé de préparation d'un composé absorbant les odeurs pour des réfrigérateurs suivant les revendications 1 à 3, **caractérisé en ce qu'**il comporte les stades suivants :
- on mélange du gel de carrageenan dans de l'eau déminéralisée à la température ambiante jusqu'à dispersion totale de l'agent gélifiant ;
- on porte à la température du mélange entre 60 et 65°C ;
- on ajoute le mélange d'isothiozaolinone ;
- on ajoute éventuellement des colorants ;
- on prépare un mélange d'huile de ricin hydrogénée éthoxylée à 40 oxyde d'éthylène et de ricinoléate de zinc dans un mélangeur distinct ;
- on ajoute ce mélange au mélange préparé et chauffé au préalable ;
- on ajoute du séquestrant ;
- on ajoute éventuellement un produit anti-ingestion.

5. Absorbant des odeurs pour réfrigérateurs, **caractérisé en ce qu'**il comprend une composition suivant l'une quelconque des revendications précédentes 1 à 3 contenue dans un récipient qui est au moins partiellement transparent de manière à permettre de contrôler la quantité restante du produit.
